(19) **Europäisches Patentamt**
**European Patent Office**
**Office européen des brevets**

(11) **EP 3 595 800 B1**

(12) **EUROPÄISCHE PATENTSCHRIFT**

(45) Veröffentlichungstag und Bekanntmachung des Hinweises auf die Patenterteilung:
**26.04.2023   Patentblatt 2023/17**

(21) Anmeldenummer: **18720105.8**

(22) Anmeldetag: **16.03.2018**

(51) Internationale Patentklassifikation (IPC):
**B01D 61/24** *(2006.01)*   **B01D 69/02** *(2006.01)*
**B01D 69/08** *(2006.01)*   **A61M 1/16** *(2006.01)*
**B01D 63/02** *(2006.01)*   **B01D 71/68** *(2006.01)*

(52) Gemeinsame Patentklassifikation (CPC):
**B01D 61/243; B01D 63/02; B01D 69/084;**
**B01D 71/68;** A61M 1/16

(86) Internationale Anmeldenummer:
**PCT/EP2018/056689**

(87) Internationale Veröffentlichungsnummer:
**WO 2018/167280 (20.09.2018 Gazette 2018/38)**

(54) **HOHLFASERMEMBRAN MIT VERBESSERTEN DIFFUSIONSEIGENSCHAFTEN**

HOLLOW FIBER MEMBRANE HAVING IMPROVED DIFFUSION PROPERTIES

MEMBRANE À FIBRES CREUSES POURVUE DE PROPRIÉTÉS DE DIFFUSION AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2017   DE 102017204524**

(43) Veröffentlichungstag der Anmeldung:
**22.01.2020   Patentblatt 2020/04**

(73) Patentinhaber: **Fresenius Medical Care Deutschland GmbH**
**61352 Bad Homburg (DE)**

(72) Erfinder:
• **KELLER, Torsten**
**66606 St. Wendel (DE)**

• **SANDER, Roland**
**66606 St. Wendel (DE)**
• **RAIKO, Igor**
**66649 Oberthal (DE)**
• **FINKLER, Christian**
**66620 Nonnweiler (DE)**

(74) Vertreter: **Ricker, Mathias**
**Wallinger Ricker Schlotter Tostmann**
**Patent- und Rechtsanwälte Partnerschaft mbB**
**Zweibrückenstrasse 5-7**
**80331 München (DE)**

(56) Entgegenhaltungen:
**EP-A1- 2 659 914     JP-A- S57 194 007**
**JP-A- 2006 051 094     JP-A- 2010 036 127**

EP 3 595 800 B1

## Beschreibung

### Thema der Erfindung

Die Erfindung betrifft eine wellenförmige Hohlfasermembran mit reduzierter Wandstärke. Ferner betrifft die Erfindung ein Verfahren zur Herstellung einer wellenförmigen Hohlfasermembran mit geringer Wandstärke. Weiterhin betrifft die Erfindung einen Hohlfasermembrandialysefilter, der eine Vielzahl von Hohlfasermembranen mit geringer Wandstärke aufweist. Weiter betrifft die Erfindung auch die Herstellung einer Hohlfasermembran und eines Hohlfasermembrandialysefilters, aufweisend eine Vielzahl von wellenförmigen Hohlfasermembranen mit geringer Wandstärke.

### Hintergrund der Erfindung

Hohlfasermembranen finden weitläufig in der Filtration von Flüssigkeiten Anwendung. In der Medizintechnik werden Hohlfasermembranen insbesondere in verschiedenen Therapieformen der extrakorporalen Blutbehandlung verwendet. In der extrakorporalen Blutbehandlung, insbesondere bei der Hämodialyse, werden urämischtoxische Blutbestandteile an einem Hohlfasermembrandialysefilter entfernt. Dabei wird im Allgemeinen von Patienten entnommenes Blut durch den Hohlraum der Hohlfasermembranen des Hohlfasermembrandialysefilters geleitet, während auf der Außenseite der Hohlfasermembran je nach Therapieform oder Art der extrakorporalen Blutbehandlung eine wässrige Flüssigkeit entlang geführt wird, die die abgetrennten Blutbestandteile aufnimmt und aus dem Hohlfasermembrandialysefilter abführt. Ein Hohlfasermembrandialysefilter weist somit insgesamt 2 Zu- und 2 Abgänge auf. Innen- und Außenseite der Hohlfasermembranen werden entsprechend diesem Prinzip der extrakorporalen Blutbehandlung auch als Blutseite und Dialysatseite bezeichnet. Die Wandungen der Hohlfasermembranen sind dabei als poröse Membran ausgestaltet, so dass durch die Membranwand der Hohlfasermembran ein Stofftransport von Blutbestandteilen von der Blutseite auf die Dialysatseite der Hohlfasermembran stattfinden kann. Der vorwiegende Transportmechanismus niedermolekularer urämischer Toxine wie beispielsweise Harnstoff ist überwiegend durch diffusive Vorgänge bestimmt, während Toxine mit mittlerem Molekulargewicht bis zu 50 kDa über konvektive Vorgänge abgetrennt werden, wozu im Betrieb des Dialysators eine sogenannte transmembrane Druckdifferenz angelegt werden muss.

Die Trennleistung, mit der toxische Blutbestandteile aus dem Blut abgetrennt werden können, wird als Clearance bezeichnet. Die Bestimmung der Clearance ist z.B. in der der Norm DIN/ EN/ ISO 8637:2014 definiert und anhand der dort beschriebenen Methode an Hohlfasermembrandialysefiltern zu ermitteln. Entsprechend wird die gemessene Clearance, neben den Parametern der Versuchsdurchführung, insbesondere durch die Eigenschaften der Hohlfasermembranen und der daraus gefertigten Hohlfasermembrandialysefilter beeinflusst. Insbesondere spielen hierbei die Porenbeschaffenheit und die Geometrie der Hohlfasermembran, sowie auch die Geometrie des Hohlfasermembrandialysefilters eine Rolle. Soll die charakteristische Abtrennleistung einer Hohlfasermembran bestimmt werden, so kann dies nur an einem geometrisch definiert aufgebauten Hohlfasermembranfilter geschehen. Auch die Flussbedingungen der Testflüssigkeiten müssen zur Charakterisierung der Membran angegeben werden. Als charakteristisches urämisches Toxin mit niedrigstem Molekulargewicht wird für die Clearance- Messungen normgemäß häufig Harnstoff verwendet. Es hat sich jedoch gezeigt, dass für Harnstoff ähnliche Clearance- Werte ermittelt werden wie für Natrium- Ionen. Die Clearance- Bestimmung mit Hilfe von Natriumionen gestaltet sich einfacher und sicherer.

Um die extrakorporale Blutbehandlungen zu verbessern, ist es ein anhaltendes Ziel in der Entwicklung von Hämodialysatoren, die Clearance zu verbessern. Im Stand der Technik wird z.B. diesbezüglich vorgeschlagen, Hohlfasermembranen wellenförmig auszugestalten. Die Wellenform ist dabei von zwei Parametern abhängig, der Wellenlänge und der Amplitude. Die Wellenform wird durch geeignete Ondulationswerkzeuge auf die Hohlfasermembranen typischerweise durch mechanische Einwirkungen aufgebracht. Die Wellenform verursacht dabei, dass die Hohlfasermembranen, die in einem Hohlfasermembrandialysefilter in einer dichten Packung vorliegen, voneinander beabstandet werden. Dadurch wird verhindert, dass die Hohlfasermembranen sich aneinander anlegen, wodurch diese besser von dem Dialysat im Hohlfasermembrandialysefilter angeströmt werden können. Dies erhöht den transmembranen Stoffübergang und damit auch die Clearance der abzutrennenden Blutbestandteile.

Weiterhin ist bekannt, die Clearance von Hohlfasermembranen durch eine Reduzierung der Wandstärke zu verbessern. Eine Reduzierung der Wandstärke bewirkt dabei eine Verkürzung des Diffusionsweges, den ein abzutrennender niedermolekularer Blutbestandteil zurücklege muss, um die Membranwand passieren zu können und korreliert damit mit einer Verringerung des Diffusionswiderstands. Im Stand der Technik sind Hohlfasermembranen für die extrakorporale Blutbehandlung bekannt, die auf Cellulose/ Cellulosederivaten basieren und eine Wandstärke von weniger als 15 μm aufweisen, die sehr kompakt ausgebildet sind und sich durch eine hohe Dichte auszeichnen. Derzeit sind in der Medizintechnik poröse Hohlfasermembranen marktdominierend, die auf Polymeren wie Polysulfon basieren und eine übliche Wandstärke von 35 bis 45 μm aufweisen.

Es hat sich allerdings gezeigt, dass im Stand der Technik übliche Wellenformen von 5 mm und mehr zusammen mit einer reduzierten Wandstärke der Hohlfasermembranen gerade nicht zu einer Steigerung der Clearance führten, sondern vielmehr zu einer Verschlechterung. Dies ist insbesondere an hitzesterilisierten Hohlfasermembrandialysefiltern beobachtet worden. Der Grund hierzu wird der Hitzesterilisation der Hohlfasermembrandialysefilter und den damit verbundenen Materialausdehnungen und -relaxationen bei erhöhten Temperaturen zugeschrieben. Üblicherweise werden die Hohlfasermembrandialysefilter während einer Hitzesterilisation auf Temperaturen von größer 100°C erhitzt. Der Wärmeeinfluss des Hitzesterilisationsprozesses führt offenbar bei Hohlfasermembranen mit reduzierter Wandstärke zu einer stärkeren Relaxation der Wellenform, als dies bei Hohlfasermembranen mit größeren Wandstärken aber gleicher Wellenlänge und Amplitude der Fall ist. Dadurch verschlechtert sich die Anströmbarkeit der Hohlfasermembranen auf der Dialysatseite eines Hohlfasermembrandialysefilters so sehr, dass trotz des verringerten Diffusionswiderstands der geringeren Membranwandstärke ein Clearance- Abfall gegenüber einem alternativen Hohlfasermembrandialysefilter mit größerer Wandstärke zu beobachten ist. Insbesondere tritt der Effekt der Clearance-Verschlechterung auf, wenn als Medium für die Hitzesterilisierung Wasserdampf verwendet wird. Durch die Temperatureinwirkung und die Wasserexposition wird der Relaxationseffekt verstärkt.

Demgemäß erweisen sich wellenförmige Hohlfasermembranen mit geringeren Wandstärken gegenüber solchen mit größeren Wandstärken als thermoinstabil. Demzufolge waren auch bisher Versuche erfolglos geblieben, hitzesterilisierbare Hohlfasermembrandialysefilter zu bauen, die eine verbesserte Clearance aufgrund der Verwendung von wellenförmigen Hohlfasermembranen mit reduzierter Wandstärke aufweisen.

Im Stand der Technik sind Hohlfasermembranen mit Wellenformen beschrieben. Die EP 1 685 862 A1 beschreibt eine Hohlfasermembran auf Basis von Polysulfon und Polyvinlypyrrolidon mit einer Wandstärke von 45 $\mu$m, einem Wellenlängenbereich von 2 bis 20 mm und einer Amplitude von 0,1 bis 5 mm. Bevorzugte Wellenlängen liegen jedoch über 4 mm bis 8 mm und bevorzugte Amplituden sind 0,2 bis 1 mm. Hohlfasermembranen mit derartigen Wandstärken und mit niedrigen Wellenlängenbereich von 2 mm sind als thermoinstabil einzustufen.

Die EP 1 671 695 A1 beschreibt einen Hohlfasermembrandialysefilter mit wellenförmigen Hohlfasermembranen, deren Wellenlänge sich aus Parametern der Hohlfasermembrangeometrie und der Gehäusegeometrie ableitet. Es wird eine rechnerische Wellenlänge von 1,4 bis 13,1 mm angegeben, wobei praxisrelevante Wellenlängen von 4 bis 12 mm angegeben werden. Bei vorgegebenen Geometrieparametern für Hohlfasermembran und Filtergehäuse wird durch die Wellenform der Hohlfasermembran eine besondere Raumausfüllung des Filtergehäuses bewirkt, wodurch ein vorteilhafter Befüllprozess des Hohlfasermembrandialysefilters durch Flüssigkeiten vor der erstmaligen Benutzung ermöglicht wird.

Die EP 2 253 371 A1 beschreibt eine wellenförmige Hohlfasermembran mit einer Wandstärke von 25 bis 45 $\mu$m, bevorzugt 35 $\mu$m, die eine Wellenlänge von 5 bis 10 mm und eine Amplitude zwischen 0,1 und 0,5 mm aufweisen kann. JPS57-194007 offenbart einen Blutfiltrations-Apparat welcher wellenförmige Hohlfasermembranen mit einer bevorzugten Wandstärke von 2-200 m, einer Wellenlänge von 0.5-50 mm und einer Amplitude von 0.05-3 mm enthält. Gemäß eines Ausführungsbeispiels werden die Werte von 22 $\mu$m, 5 mm und 0.8 mm für die Wandstärke, Wellenlänge und Amplitude offenbart. Polysulfon ist als geeignetes Polymer offenbart. EP 2 659 914 A1 offenbart die Verwendung von wellenförmigen Hohlfasermembranen in Dialysegeräten, wobei die Hohlfasermembranen eine bevorzugte Wandstärke von 20-70 $\mu$m, eine Wellenlänge von 5-10 mm und eine Amplitude von 0.1-1.5 mm aufweisen. JP 2010-036127 beschreibt ein Hohlfasermembranmodul bestehend aus Hohlfasermembranen, auf die eine Wellenform aufgebracht wird wonach die Hohlfasermembranen an Stäben auf der äußeren Umfangsfläche von Walzen entlanglaufen und dabei in einem Eingriffsteil zwischen einem Paar von Walzen eingeklemmt werden, wobei ein Durchschnittswert der Harnstoff-Clearance in einem wässrigen Lösungssystem, gemessen unter Verwendung von fünf Hohlfasermembranmodulen, 170 ml/min/m$^2$ oder mehr beträgt und die Standardabweichung 10.0 oder weniger beträgt.

JP 2006-051094 beschreibt eine gewellte Hohlfasermembran mit einem Innendurchmesser von 190-250 $\mu$m, einer Membrandicke von 10-60 $\mu$m und einer Dickenabweichung von $\geq$ 0,6. Die Hohlfasermembran hat eine dichte Schicht auf ihrer inneren Oberfläche, eine Wellenform, deren Wellenlänge $\geq$ 10 mm beträgt, und eine Amplitude die $\geq$ 0,6 mm beträgt.

## Aufgabe der Erfindung

Bisher bekannte Techniken zur Clearancesteigerung von hitzesterilisierbaren Hohlfasermembranen durch eine wellenförmige Ausgestaltung der Hohlfasermembranen sind nicht für Hohlfasermembranen mit geringer Wandstärke anwendbar. Es war daher ein Ziel der vorliegenden Erfindung eine wellenförmige Hohlfasermembran mit geringer Wandstärke bereitzustellen, die ausreichend thermostabil ist und somit die Leistungsdaten auch nach einer Hitzesterilisierung weitgehend behält.

Damit verbunden war es ein weiteres Ziel der Erfindung, ein Verfahren bereitzustellen, mit dem eine derartige thermostabile Hohlfasermembran hergestellt werden kann.

Ein weiteres Ziel der Erfindung bestand darin, einen hitzesterilisierten Hohlfasermembrandialysefilter mit hohen Leistungsdaten für die Filtration bereitzustellen, der Hohlfasermembranen mit geringer Wandstärke aufweist.

Damit verbunden war es ein weiteres Ziel der Erfindung ein Verfahren bereitzustellen mit dem derartige Hohlfasermembrandialysefilter, die wellenförmige Hohlfasermembranen mit geringer Wandstärke aufweisen, hergestellt werden können.

**Zusammenfassung der Erfindung**

In einem ersten Aspekt betrifft die Erfindung eine wellenförmige Hohlfasermembran für die extrakorporale Blutbehandlung, umfassend wenigstens ein erstes hydrophobes Polymer, das ausgewählt ist aus der Gruppe der Polyarylether (Polysulfone, Polyarylketone, Polyetherketone), Polyamide, Polyester, Polycarbonate, Polyacrylate und -methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere, und wenigstens ein zweites hydrophiles Polymer, das ausgewählt ist aus der Gruppe der Polyvinylpyrrolidone oder Polyethylenglykole oder Mischungen hieraus,

mit einer Wandstärke (w) von 20 $\mu$m oder mehr und 30 $\mu$m oder weniger,
einer Amplitude (a) der Wellenform im Bereich von 0,005 bis 0,15 mm, insbesondere von 0,01 bis 0,12 mm, und
einem Lumendurchmesser (I) von 160 bis 230 $\mu$m,
dadurch gekennzeichnet,
dass die Wellenform der Hohlfasermembran eine Wellenlänge ($\lambda$) im Bereich von mehr als 1 mm bis 4 mm oder mehr als 1 mm und weniger als 4mm aufweist,
sowie dadurch gekennzeichnet, dass die Porosität der Hohlfasermembran größer als 65% und kleiner als 78% ist, wobei die Porosität, wie hierin beschrieben, bestimmt wird.

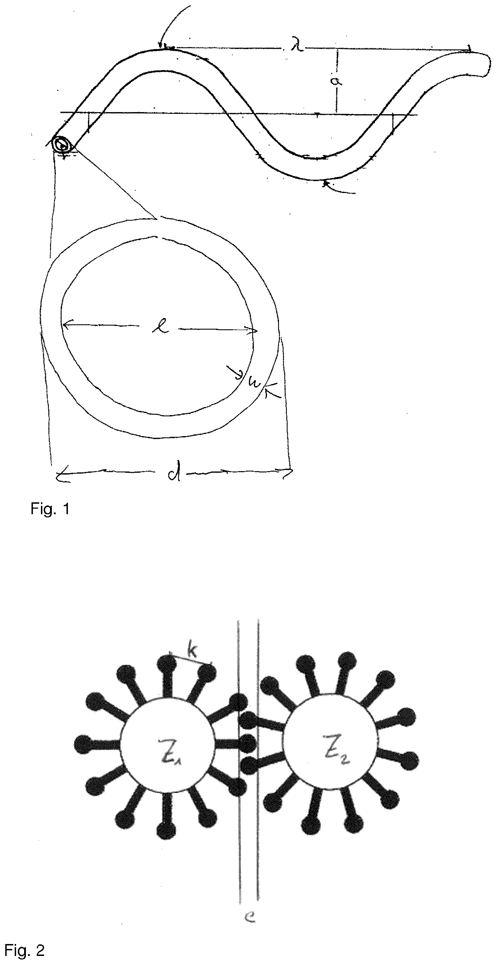

In einer weiteren Ausführungsform gemäß dem ersten Aspekt der Erfindung ist die wellenförmige Hohlfasermembran dadurch gekennzeichnet, dass die Wellenform im Wesentlichen sinusförmig ist.
In einer weiteren Ausführungsform gemäß dem ersten Aspekt der Erfindung ist die wellenförmige Hohlfasermembran dadurch gekennzeichnet, dass die Porosität der Hohlfasermembran größer als 70%, weiter insbesondere größer als 72 % ist.
Typischerweise handelt es sich bei der Welle der erfindungsgemäßen Hohlfasermembran um eine Longitudinalwelle, wie sie auch in der Ausführungsform der Fig. 1 dargestellt ist.
In einem zweiten Aspekt betrifft die Erfindung ein Verfahren zur Herstellung einer wellenförmigen Hohlfasermembran für die extrakorporale Blutbehandlung, umfassend wenigstens ein erstes hydrophobes Polymer, umfassend wenigstens ein erstes hydrophobes Polymer, das ausgewählt aus der Gruppe der Polyarylether (Polysulfone Polyarylketone, Polyetherketone), Polyamide, Polyester, Polycarbonate, Polyacrylate und- methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere und wenigstens ein zweites hydrophiles Polymer, welches ausgewählt ist aus der Gruppe der Polyvinylpyrrolidone oder der Polyethylenglykole oder Mischungen hieraus, aufweisend die Schritte:

Herstellen einer Hohlfasermembran mit einer Wandstärke von 20 $\mu$m oder mehr und 30 $\mu$m oder weniger, einer Porosität, die größer als 65% und kleiner als 78% ist, wobei die Porosität wie in der Beschreibung definiert, bestimmt wird. und einem Lumendurchmesser (I) von 160 bis 230 $\mu$m aus einer Spinnmasse durch einen Spinn- und Phaseninversionsprozess,
Bereitstellen wenigstens eines Ondulationswerkzeugs, gegebenenfalls Ausrichten des Ondulationswerkzeugs, zur Erzeugung einer Wellenprägung mit vorbestimmter Wellenlänge und Amplitude,
Behandeln der Hohlfasermembran mit dem Ondulationswerkzeug, so dass eine Wellenlänge der Ondulation von mehr als 1 mm bis 4mm, oder von mehr als 1mm und weniger als 4 mm und eine Amplitude (a) im Bereich von 0,005 bis 0,15 mm, insbesondere von 0,01 bis 0,12 mm, resultiert.

In einer weiteren Ausführungsform gemäß dem zweiten Aspekt der Erfindung ist das Verfahren zur Herstellung einer wellenförmigen Hohlfasermembran dadurch gekennzeichnet, dass das Ondulationswerkzeug zwei ineinandergreifende Zahnräder aufweist, zwischen denen die Hohlfasermembran hindurchgeführt wird.
In einer weiteren Ausführungsform gemäß dem zweiten Aspekt der Erfindung ist das Verfahren zur Herstellung einer wellenförmigen Hohlfasermembran dadurch gekennzeichnet, dass die Zahnräder des Ondulationswerkzeugs einen Kopfabstand der Zähne von mehr als 1 mm und weniger als 5 mm, insbesondere weniger als 4 mm, aufweisen.
In einer weiteren Ausführungsform gemäß dem zweiten Aspekt der Erfindung ist das Verfahren zur Herstellung einer wellenförmigen Hohlfasermembran dadurch gekennzeichnet, dass die Eingriffstiefe der Zähne der Zahnräder des Ondulationswerkzeugs 0,1 bis 0,5 mm, insbesondere 0,1 bis 0,2 mm beträgt.
In einer weiteren Ausführungsform gemäß dem zweiten Aspekt der Erfindung ist das Verfahren zur Herstellung einer wellenförmigen Hohlfasermembran dadurch gekennzeichnet, dass die Spinnmasse wenigstens ein erstes hydrophobes Polymer umfasst, welches ausgewählt ist aus der Gruppe der Polyarylether (Polysulfone, Polyarylketone, Polyetherketone), Polyamide, Polyester, Polycarbonate, Polyacrylate und -methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere, und dass die Spinnmasse wenigstens ein zweites hydrophiles Polymer umfasst, welches ausgewählt ist aus der Gruppe der Polyvinylpyrrolidone oder Polyethylenglykole oder Mischungen hieraus, und wenigstens ein Lösungsmittel, insbesondere N-Methylpyrrolidon, N,N Dimethyl acetamid, N,N-Diemthylformamid oder Dimethylsulfoxid aufweist.

In einem dritten Aspekt betrifft die Erfindung einen Hohlfasermembrandialysefilter, der eine Vielzahl von wellenförmigen Hohlfasermembranen gemäß einer Ausführungsform nach dem ersten Aspekt der Erfindung oder hergestellt nach wenigstens einer Ausführungsform gemäß dem zweiten Aspekt der Erfindung aufweist.

In einem vierten Aspekt der Erfindung betrifft die Erfindung ein Verfahren zur Herstellung eines Hohlfasermembrandialysefilters nach dem dritten Aspekt der Erfindung, wobei das Verfahren die folgenden Schritte aufweist:

- Bereitstellen eines Hohlfasermembranbündels, aufweisend eine Vielzahl von Hohlfasermembranen nach wenigstens einer Ausführungsform gemäß des ersten Aspekts der Erfindung oder erhältlich nach wenigstens einer Ausführungsform gemäß des zweiten Aspekts der Erfindung
- Bereitstellen eines Filtergehäuses,
- Einbringen des Hohlfasermembranbündels in das Filtergehäuse und Vergießen der Enden der Hohlfasermembranen in dem Filtergehäuse mit einer Vergussmasse,
- Wiederöffnen der vergossenen Membranenden, so dass ein Fluss durch die Lumen des überwiegenden Teils der Hohlfasern ermöglicht ist, und Endmontieren des Dialysefilters,
- Sterilisieren des Hohlfasermembrandialysefilters durch ein Hitzesterilisationsverfahren.

In einer Ausführungsform gemäß des vierten Aspekts ist das Verfahren zur Herstellung eines Hohlfasermembrandialysefilter dadurch gekennzeichnet, dass das Hitze-sterilisationsverfahren einen Schritt umfasst, bei dem der Hohlfasermembrandialysefilter mit auf 100 bis 150°C erhitztem Wasser oder Wasserdampf durchspült wird.

In einer Ausführungsform gemäß des vierten Aspekts ist das Verfahren zur Herstellung eines Hohlfasermembrandialysefilter dadurch gekennzeichnet, dass die Behandlung mit Wasser oder Wasserdampf wenigstens einen Schritt umfasst, bei dem Wasser oder Wasserdampf in das Innere der Hohlfasermembranen geleitet wird und durch Druckbeaufschlagung durch die Membranwand auf die Außenseite der Fasern permeiert wird.

In einer Ausführungsform gemäß des vierten Aspekts ist das Verfahren zur Herstellung eines Hohlfasermembrandialysefilter dadurch gekennzeichnet, dass die Hohlfasermembranen bei einer Temperatur von 100°C bis 150°C getrocknet werden.

**Kurzbeschreibung der Figuren und der Tabelle**

Fig. 1 zeigt eine schematische Darstellung einer wellenförmigen Hohlfasermembran gemäß der Erfindung. In Fig1 sind bezeichnet:

λ    die Wellenlänge
a    die Amplitude
d    der Durchmesser
l    der Lumendurchmesser
w    die Wandstärke

Fig. 2 zeigt in schematischer Darstellung ein Ondulationswerkzeug mit zwei Zahnrädern. in Fig. 2 sind bezeichnet:

$Z_1$    erstes Zahnrad
$Z_2$    zweites Zahnrad
k    Kopfabstand zweier benachbarten Zähne eines Zahnrades.
e    Eingriffstiefe der beiden Zahnräder

Tabelle 1 zeigt die Natrium Clearance von erfindungsgemäßen Hohlfasermembranen in Abhängigkeit verschiedener Wellenlängen vor und nach der Hitzesterilisierung.

**Detaillierte Beschreibung der Erfindung**

In einem ersten Aspekt betrifft die Erfindung die zuvor genannte wellenförmige Hohlfasermembran.

Überraschenderweise wurde im Rahmen der experimentellen Erarbeitung der Erfindung festgestellt, dass durch eine erfindungsgemäße wellenförmige Hohlfasermembran eine thermostabile Hohlfasermembran erhalten werden kann. Insbesondere hat sich erwiesen, dass der Abfall der Clearance durch einen Hitzesterilisationsverfahren nicht so stark ausgeprägt ist. Insbesondere ist bei der erfindungsgemäßen Hohlfasermembran der Abfall der Clearance durch ein Hitzesterilisationsverfahren so gering, dass insgesamt ein Anstieg der Clearance durch die verminderte Wandstärke im Vergleich zu einer Membran mit 35 $\mu$m oder mehr beobachtet werden kann.

Als eine *"wellenförmige"* Hohlfasermembran wird im Sinne der vorliegenden Anmeldung eine Hohlfasermembran verstanden, die entlang ihrer Längsausdehnung zumindest teilweise konkave und konvexe Bereiche aufweist. Die konkaven und konvexen Bereiche sind entlang der Längsausdehnung der Hohlfasermembran unregelmäßig oder bevorzugt regelmäßig, also periodisch, angeordnet. Die konkaven und konvexen Bereiche folgen abwechselnd aufeinander und können gegebenenfalls voneinander beabstandet sein durch einen Bereich, der nicht konkav oder konvex ausgestaltet ist. Die Maxima der konkaven und konvexen Bereiche werden im Sinne der vorliegenden Anmeldung auch als *"Amplituden"* bezeichnet. Weiterhin werden im Sinne der vorliegenden Anmeldung verschiedene Ausgestaltungen der erfindungsgemäßen Hohlfasermembran als wellenförmig bezeichnet. Insbesondere wird auch eine Zick-Zack- Ausgestaltung oder eine Sägezahnausgestaltung oder bevorzugt eine sinusförmige Ausgestaltung als wellenförmig bezeichnet.

Der Begriff der *"Wellenlänge"* bedeutet im Sinne der vorliegenden Anmeldung jener Abschnitt in Längsausrichtung der Hohlfasermembran, der dem Abstand von einer Wellenamplitude zur übernächsten Wellenamplituden entspricht. Dies ist in Fig. 1 gezeigt. In Fig 1 ist die Wellenlänge mit $\lambda$ bezeichnet. Es können Wellenlängen vorliegen, die entlang der Längsausrichtung der Hohlfasermembran konstant sind oder variieren. Bevorzugt sind konstante Wellenlängen.

Der Begriff *"thermostabil"* bedeutet dabei, dass unter Hitzebedingungen die Leistungsdaten, insbesondere die Clearance, der erfindungsgemäßen Hohlfasermembran weitgehend erhalten bleiben. Es hat sich gezeigt, dass die Amplitude im Verlaufe der Hitzesterilisierung unter Erhalt der Wellenlänge abnimmt. Werden jedoch bei erfindungsgemäßen Hohlfasermembranen mit Wandstärken größer als 20 $\mu$m und kleiner als 30 $\mu$m geringe Wellenlängen angewendet, die größer als 1 mm, aber kleiner als 5 mm sind, so kommt es nach der Hitzesterilisierung nur zu einem geringen Abfall der Clearance, so dass die besonders positiven Eigenschaften, die aus der geringen Wandstärke resultieren, auch nach der Hitzestabilisierung erhalten bleiben. Besonders ausgeprägt ist der Effekt, wenn die Wellenlänge weniger als 4 mm beträgt.

Insbesondere wird unter dem Begriff auch verstanden, dass eine wellenförmige Hohlfasermembran unter Bedingungen der Hitzesterilisation von oberhalb 100°C sterilisiert werden kann, ohne dass der positive Effekt der Wellenform der Hohlfasermembran im erheblichen Maße verloren geht. Die Stabilität der wellenförmigen Hohlfasermembranen unter Hitzebedingungen wird, wie beschrieben, vorliegend durch die Veränderung der Clearance der Hohlfasermembran durch ein Hitzesterilisationsverfahren bewertet. Demnach kann eine erfindungsgemäße wellenförmige Hohlfasermembran als thermo-stabil bezeichnet werden, wenn die Clearance durch den Schritt der Hitzesterilisation nicht zu stark absinkt. Ein Absinken der Natrium- Clearance von weniger als 13 ml/min, insbesondere von 12 ml/min oder weniger als 12 ml/min, insbesondere von 10 ml/min oder weniger als 10 ml/min, insbesondere 8 ml/min oder weniger als 8ml/min hat sich als vorteilhaft erwiesen. Entsprechende Hohlfasermembranen erweisen sich als vorteilhaft thermostabil.

In weiteren Ausführungsformen kann die Wellenlänge der Hohlfasermembran mindestens 1,5 mm, oder mindestens 2 mm, oder maximal 3,5 mm oder maximal 3 mm aufweisen. In bestimmten Ausführungsformen beträgt die Wellenlänge 1,5 mm und maximal 4 mm, weiter bevorzugt mindestens 2 mm und maximal 3,5 mm, weiter bevorzugt mindestens 2 mm und maximal 3 mm.

Generell sind kürzere Wellenlängen bevorzugt, da bei kürzeren Wellenlängen eine größere Thermostabilität und ein geringerer Verlust der Clearance beobachtet wird. Eine untere Grenze der Wellenlänge ist abhängig von der Knickstabiltät der Hohlfasermembran, die wiederum abhängig ist vom verwendeten Material, der Wandstärke und der Amplitude der Wellenform. Bei längeren Wellenlängen von 5 mm oder mehr sind die wellenförmigen Hohlfasermembranen mit einer Wandstärke unterhalb von 35 $\mu$m nicht ausreichend thermostabil, so dass kein positiver Clearance-Effekt durch die reduzierte Wandstärke beobachtet werden kann.

In weiteren Ausführungsformen kann die Wandstärke der wellenförmigen Hohlfasermembranen mindestens 22 $\mu$m und/oder maximal 28 $\mu$m betragen.

Hohlfasermembranen mit reduzierter Wandstärke, insbesondere unterhalb einer Wandstärke von 30 $\mu$m besitzen eine bessere Ausformbarkeit als Hohlfasermembranen mit größerer Wandstärke. Das bedeutet, dass sich an Hohlfasermembranen mit reduzierter Wandstärken kleinere Wellenlängen durch sogenannte Ondulationswerkzeuge aufbringen lassen als bei Hohlfasermembranen mit größerer Wandstärke, ohne dass die Hohlfasermembranen durch Knicke oder Faltungen geschädigt werden.

Als *"Ondulationswerkzeug"* wird im Sinne der vorliegenden Anmeldung eine Vorrichtung verstanden, mit der eine *"Ondulation"* also eine Wellenform auf eine lineare Hohlfasermembran aufgebracht werden kann. Die *"Wellenform"* einer Hohlfasermembran ist durch ihre wellenförmige Ausgestaltung, wie in Absatz [0034] beschrieben, definiert. Generell wird im Herstellprozess der Hohlfasermembranen die Wellenform auf die Hohlfasermembran durch mechanische Einwirkungen bewirkt. Fig. 2 zeigt ein typisches Ondulationswerkzeug in schematischer Darstellung.

Generell kann jede Ausbiegung der wellenförmigen Hohlfasermembran als erfindungsgemäß aufgefasst werden, unabhängig von der speziellen Wellenform die die erfindungsgemäße Hohlfasermembran beschreibt. In diesem Sinne können auch zick-zack-artige oder sägezahn-artige Wellenformen mit dem Begriff der Amplitude beschrieben werden. Eine erfindungsgemäße Form der Ausbiegung ist periodisch. Am Beispiel einer periodischen Welle wird die Amplitude im Sinne der vorliegenden Anmeldung als die Hälfte des horizontalen Abstandes zwischen einem Wellental und einem Wellenberg verstanden Dies ist in Fig. 1 gezeigt. In Fig. 1 ist die Amplitude mit "a" bezeichnet. *"Wellental"* und *"Wellenberg"* sind dabei als Bezeichnung für zwei benachbarte Biegungen der Hohlfasermembran zu verstehen. Die periodische Welle der Fig. 1 ist beispielhaft sinusförmig.

Die Amplitude der erfindungsgemäßen wellenförmigen Hohlfasermembran kann mindestens 0,01 mm oder mindestens 0,02 mm oder maximal 0,12 oder maximal 0,1 mm betragen. In einer Ausführungsform beträgt die Amplitude der wellenförmigen Hohlfasermembranen mindestens 0,02 mm bis maximal 0,12 mm, oder bevorzugt mindestens 0,04 mm bis 0,12 mm.

Generell ist das Aufbringen von Wellenformen auf Hohlfasermembranen mit großer Amplitude bei gegebener Wandstärke und Wellenlänge durch die Knickstabilität der Hohlfasermembran beschränkt. Große Amplituden sind jedoch im Hinblick auf die Trennleistung erwünscht, weil sie eine bessere Beabstandung der Hohlfasermembran im Hohlfasermembranbündel bzw. im Hohlfasermembrandialysefilter bewirken. Die Beabstandung der Hohlfasermembranen im Hohlfasermembrandialysefilter bewirkt eine bessere Anströmbarkeit der Hohlfasermembranen durch Dialysierflüssigkeit und damit auch eine bessere Clearance. Dagegen ist bei wellenförmigen Hohlfasermembranen mit kleiner Amplitude prinzipiell die Beabstandungstendenz der Hohlfasermembranen und damit auch die Anströmbarkeit mit Dialysierflüssigkeit im Hohlfasermembrandialysefilter reduziert. Überraschenderweise zeigte sich jedoch, dass bei Hohlfasermembranen mit geringer Wandstärke in Verbindung mit einer geringen Wellenlänge jeweils im erfindungsgemäßen Bereich eine kleine Amplitude bereits sehr gute Clearance- Werten und einen geringen Clearance- Abfall ermöglicht. Durch die vergleichsweise geringen Amplituden kann zudem eine besonders schonende extrakorporale Behandlung des Blutes gewährleistet werden.

Die erfindungsgemäße Hohlfasermembran weist einen Lumendurchmesser von 160 bis 230 $\mu$m auf. Der Begriff *"Lumendurchmesser"* bezeichnet die lichte Weite des Innenraums der Hohlfasermembran, der an einem Querschnitt senkrecht zur Längsausrichtung der Hohlfasermembran gemessen wird. Dies ist in Fig 1. gezeigt.

Die erfindungsgemäße Hohlfasermembran weist einen Lumendurchmesser von mindestens 160 $\mu$m, oder mindestens 170 $\mu$m oder mindestens 180 $\mu$m, oder maximal 230 $\mu$m, oder maximal 210 $\mu$m, oder maximal 200 $\mu$m auf. In einer Ausführungsform beträgt der Lumendurchmesser mindestens 170 $\mu$m bis maximal 230 $\mu$m, bevorzugt 180 $\mu$m bis maximal 230 $\mu$m.

Der Lumendurchmesser einer Hohlfasermembran steht mit der Stabilität der Hohlfasermembran im Zusammenhang. Insbesondere wird die Stabilität der Hohlfasermembran bei Hohlfasermembranen mit reduzierter Wandstärke von unterhalb 31 $\mu$m negativ beeinträchtigt. Eine Hohlfasermembran mit großem Lumendurchmesser neigt dazu, durch mechanische Einwirkungen deformiert oder beschädigt zu werden. Dies kann insbesondere der Fall sein, wenn eine Hohlfasermembran mit einem Ondulationswerkzeug behandelt wird, um eine Wellenform zu erzeugen. Geringere Lumendurchmesser erhöhen dagegen die Stabilität von Hohlfasermembranen mit geringen Wandstärken gegenüber mechanischen Einwirkungen. Eine untere Grenze des Lumendurchmessers bedingt sich durch die Verwendung der Hohlfasermembran für die Blutbehandlung. Lumendurchmesser von unterhalb 160 $\mu$m erweisen sich aus therapeutischer Sicht nicht als praktikabel. Bevorzugt sind Lumendurchmesser von mindestens 180 $\mu$m, weil bei solchen Ausführungen eine besonders schonende Blutbehandlung möglich ist.

In einer Ausführungsform gemäß des ersten Aspekts der Erfindung ist die wellenförmige Hohlfasermembran dadurch gekennzeichnet, dass die Wellenform im Wesentlichen sinusförmig, insbesondere sinusförmig, ist.

Mit der Bezeichnung einer wellenförmigen Hohlfasermembran mit *"sinusförmiger"* Wellenform ist im Zusammenhang der vorliegenden Anmeldung zu verstehen, dass die konkaven und konvexen Bereiche der erfindungsgemäßen Hohlfasermembran entlang der Längsausdehnung gleichförmig und regelmäßig sind, d.h. eine im Wesentlichen konstante Wellenlänge und konstante Amplitude aufweisen und der Form einer Sinusfunktion im mathematischen Sinne angenähert sind.

Derartige sinusförmige Wellenformen von Hohlfasermembranen sind in der Herstellung vorteilhaft. Weiterhin ist die Beabstandung der Hohlfasermembranen in einem Hohlfasermembrandialysefilter oder einem Hohlfasermembranbündel vorteilhaft.

Gemäß der Erfindung ist die erfindungsgemäße Hohlfasermembran dadurch gekennzeichnet, dass das wenigstens eine erste hydrophobe Polymer ausgewählt ist aus der Gruppe der Polyarylether (Polysulfone, Polyarylketone, Polyetherketone), Polyamide, Polyester, Polycarbonate, Polyacrylate und -methacrylate, Polymethacrylimide, Polyvinylidenfluoride, Polyimide oder Polyacrylnitrile, oder der Copolymere umfassend entsprechende Monomereinheiten der genannten Polymere oder der Mischungen der genannten Polymere.

Die erfindungsgemäße Hohlfasermembran ist dadurch gekennzeichnet, dass die Hohlfasermembran wenigstens ein zweites hydrophiles Polymer umfasst und dass das wenigstens eine zweite Polymer ausgewählt ist aus der Gruppe der Polyvinylpyrrolidone oder Polyethylenglykole oder Mischungen daraus.

Als *"hydrophobe Polymere"* werden im Sinne der vorliegenden Anmeldung solche Polymere verstanden, die nicht wasserlöslich sind. Als *"hydrophile Polymere"* werden solche Polymere verstanden, die wasserlöslich sind oder Hydrogele bilden.

Das zumindest eine hydrophobe Polymere bildet erfindungsgemäß den Hauptteil des Polymermaterials aus dem die erfindungsgemäßen Hohlfasermembranen hergestellt sind. Die Porenstruktur der erfindungsgemäßen Hohlfasermembran wird durch das zumindest eine erste hydrophobe Polymermaterial gebildet und bewirkt das Ausschluss- bzw. Trennverhalten der Hohlfasermembranen. Das mindestens eine zweite hydrophile Polymere ist in einem geringen Anteil im Membranmaterial der Hohlfasermembranen vorhanden. Das wenigstens eine zweie hydrophile Polymer ist oberflächennah auf der Hohlfasermembran abgelagert und bewirkt die Benetzbarkeit der Hohlfasermembran mit wässrigen Behandlungsflüssigkeiten, wie z.B. Blut oder Dialysierflüssigkeit. Weiterhin sind die hydrophilen Polymere ergänzend zu einem Lösemittel für die Porenbildung während der Herstellung von Hohlfasermembranen durch ein Spinnverfahren und Phaseninversionsprozess verantwortlich.

Das zumindest eine erste hydrophobe Polymer ist bevorzugt ein Polysulfon und zeichnet sich dadurch aus, dass daraus hergestellte Hohlfasermembranen besonders gut hitzesteriliserbar sind.

Unter einem Polymer mit der Bezeichnung *"Polysulfon"* wird im Zusammenhang der vorliegenden Anmeldung ein Polymer verstanden, dass mindestens eine Sulfongruppe in der Haupt- oder Nebenkette des Polymers aufweist. Typische Beispiele von Polysulfonen sind: Polysulfon (PSU), Polyethersulfon (PES), Polyphenylsulfon und Copolymere enthaltend zumindest eine Sulfongruppe. Weitere Vertreter von Polysulfonpolymeren sind im Stand der Technik bekannt und im Sinne der Erfindung für die Herstellung von Blutbehandlungsmembranen geeignet.

Die erfindungsgemäße wellenförmige Hohlfasermembran ist dadurch gekennzeichnet, dass das zumindest eine zweite hydrophile Polymer ausgewählt ist aus der Gruppe der Polyvinylpyrrolidone oder der Polyethylenglykole oder Mischungen hieraus. Die Gruppe der Polyvinylpyrrolidone ist insbesondere bevorzugt, da Polyvinylpyrrolidone hitzesterilisierbar sind und bezüglich der Herstellung der Hohlfasermembranen durch Spinnverfahren und Phaseninversionsprozess in Verbindung mit mindestens einem hydrophoben Polymer, insbesondere mit Polysulfonen, bevorzugt sind. Polyvinylpyrrolidone sind zudem im besonderen Maße hämokompatibel, d.h. dass keine oder nur geringe schädigende Wirkungen auf das Blut von dem Polymer ausgehen. Dies gilt im Besonderen im Zusammenwirken mit einem Polysulfon als hydrophobem Polymer.

Unter einem Polymer mit der Bezeichnung *"Polyvinylpyrrolidon"* wird im Sinne der vorliegenden Anmeldung ein Polymer verstanden, dass unter Verwendung des Monomeren Vinylpyrrolidon oder Derivaten davon hergestellt ist.

Die erfindungsgemäße wellenförmige Hohlfasermembran ist dadurch gekennzeichnet, dass die Porosität der Hohlfasermembran größer als 65%, insbesondere größer als 70%, weiter insbesondere größer als 72% ist. Neben der Reduzierung der Wandstärke ist eine geeignete Maßnahme die Erhöhung der Porosität, um die Diffusionseigenschaften einer Hohlfasermembran nachhaltig zu verbessern. Nachteilig bei der Erhöhung der Porosität ist jedoch wiederum, wie bei der Reduzierung der Wandstärke, dass die mechanische und thermische Stabilität herabgesetzt wird. So führt die Erhöhung der Porosität nicht zwangsläufig zu einer Verbesserung der Clearance, insbesondere wenn ein Schritt der Hitzesterilisierung durchgeführt wurde. Es werden jedoch besonders hohe Clearancewerte, auch nach der Hitzesterilisierung, erzielt, wenn Hohlfasermembranen mit geringer Wandstärke, insbesondere mit einer Wandstärke von 30 $\mu$m oder weniger, und hoher Porosität von größer 65% mit der erfindungsgemäßen wellenförmigen Ondulation versehen wird. Besonders bevorzugte Hohlfasermembranen mit hoher Porosität sind dabei im Wesentlichen frei, insbesondere vollständig frei, von sogenannten dendritischen Hohlräumen oder "makrovoids". Im Wesentlichen frei von Makrovoids bedeutet dabei, dass weniger als 5% des Wandvolumens der porösen Hohlfasermembran von solchen Makrovoids eingenommen wird.

In einer weiteren Ausführungsform ist vorgesehen, dass die Porosität der Hohlfasermembran größer als 65%, insbesondere größer als 70% und weiter insbesondere größer als 72% und kleiner als 78%, insbesondere kleiner als 76%, ist. Es hat sich gezeigt, dass Hohlfasermembranen mit geringer Wandstärke zwischen 20 und 30 $\mu$m in der Porosität besonders eingestellt werden sollten, um die gewünschten Leistungsparameter optimal zu erreichen.

Ein zweiter Aspekt der Erfindung betrifft ein Verfahren zur Herstellung einer erfindungsgemäßen Hohlfasermembran der zuvor beschriebenen Art.

Die Begriffe *"Spinnverfahren"* und *"Phaseninversionsprozess"* sind im Stand der Technik im Zusammenhang mit der Herstellung von Hohlfasermembran hinlänglich bekannt. Sie sind beispielsweise in der Anmeldung DE 102016224627.5 beschrieben. Der Offenbarungsgehalt der DE102016224627.5 ist somit Bestandteil dieser Anmeldung.

Gemäß einer Ausführungsform des zweiten Aspekts der Erfindung handelt es sich bei dem Ondulationswerkzeug um eine Zahnradanordnung, wie beispielhaft in Fig. 2 gezeigt. Fig. 2 zeigt zwei Zahnräder $Z_1$ und $Z_2$, die so angelegt

sind, dass die Zahnräder ineinandergreifen und in entgegengesetzter Rotation zueinander bewegt werden können. Insbesondere kann ein Ondulationswerkzeug gemäß dieser Ausführungsform so angelegt sein, dass die Eingriffstiefe der Zahnräder eingestellt werden kann, indem die Zahnräder aufeinander zu bzw. voneinander weg bewegt werden. Um eine Wellenform auf eine Hohlfasermembran aufzubringen, wird eine Hohlfasermembran zwischen die im Eingriff stehenden Zahnräder hindurchgeführt. Die im Eingriff stehenden Zahnräder bewirken eine Zugspannung auf die Hohlfasermembran, so dass die Hohlfasermembran in periodischen Abschnitten unterschiedlich, entsprechend der Einwirkung der eingreifenden Zähne der Zahnräder verformt wird. In der Folge entsteht durch die periodisch unterschiedliche Verformung eine wellenförmige Hohlfasermembran. Die Form der Zähne und die Eingriffstiefe ist dabei so angepasst, dass sich möglichst keine Quetschungen auf die Hohlfasermembranen auswirken, die einen deformierten Querschnitt der Hohlfasermembran verursachen.

Die Zähne der Zahnräder des Ondulationswerkzeugs weisen einen Kopfabstand von weniger als 5 mm auf.

Der Begriff *"Kopfabstand"* bezeichnet im Sinne der Vorliegenden Anmeldung den Abstand zweier benachbarter Zähne der Zahnräder. Alle Zähne eines Zahnrades sind dabei gleich voneinander beabstandet. Der Kopfabstand der Zähne bewirkt die Wellenlänge der Hohlfasermembran. Der Kopfabstand der Zähne ist in Fig. 2 mit "k" bezeichnet

Insbesondere kann der Kopfabstand mehr als 1 mm, oder mindestens 1,5 mm, oder mindestens 2 mm, oder weniger als 5 mm, oder maximal 4 mm oder maximal 3,5 mm oder maximal 3 mm aufweisen. In einer Ausführungsform beträgt der

Kopfabstand mehr als 1 mm und weniger als 5 mm, bevorzugt mindestens 1,5 mm und maximal 4 mm, weiter bevorzugt mindestens 2 mm und maximal 3,5 mm, weiter bevorzugt mindestens 2 mm und maximal 3 mm.

In einer Ausführungsform beträgt die Eingriffstiefe der Zahnräder des Ondulationswerkzeugs 0,1 bis 0,5 mm, insbesondere 0,1 bis 0,2 mm. Die Eingriffstiefe der Zahnräder bewirkt die Größe der Amplituden der wellenförmigen Hohlfasermembran. Eine geringe Eingriffstiefe bewirkt eine kleine Amplitude, wobei eine große Eingriffstiefe eine große Amplitude bewirkt. Die Eingriffstiefe der Zahnräder richtet sich nach der Größe der Amplitude, welche die erfindungsgemäße Hohlfasermembran aufweisen soll. Die resultierende Amplitude der Hohlfasermembran ist erfindungsgemäß geringer als die Eingriffstiefe der Zahnräder. Die Einbringung der Wellenform durch Zahnräder ist besonders wirtschaftlich und führt gegenüber anderen Verfahren, die im Stand der Technik bekannt sind, zu besonders gut definierten gleichmäßigen Wellenformen, insbesondere zu periodischen, weiterhin insbesondere zu sinusförmigen Wellenformen. Dies führt zu hohen Clearance- Werten.

Nach einer Weiterbildung des erfindungsgemäßen Verfahrens zur Herstellung einer erfindungsgemäßen wellenförmigen Hohlfasermembran weist die Spinnmasse wenigstens ein erstes hydrophobes Polymer, insbesondere ausgewählt aus der Gruppe der Polyarylether (Polysulfone, Polyarylketone, Polyetherketone), Polyamide, Polyester, Polycarbonate, Polyacrylate und -methacrylate, Polymethacrylimide, Polyvinylidenfluoride, Polyimide oder Polyacrylnitrile, der Copolymere umfassend entsprechende Monomereinheiten der genannten Polymere oder der Mischungen der genannten Polymere und weiterhin weist die Spinnmasse wenigstens ein zweites wenigstens hydrophiles Polymer, insbesondere ausgewählt aus der Gruppe der Polyvinylpyrrolidone oder der Polyethylenglykole oder Mischungen hieraus auf, sowie wenigstens ein Lösungsmittel, insbesondere N-Methylpyrrolidon, N,N-Dimethylacetamid, N,N-Diemthylformamid oder Dimethylsulfoxid.

Hohlfasermembranen aus den genannten Materialien erweisen sich als ausreichend stabil, um hitzesterilisierbare, thermostabile, deformationsfeste wellenförmige erfindungsgemäße Hohlfasermembranen mit reduzierter Wandstärke von 30 μm Wandstärke oder weniger herzustellen. Der Begriff *"deformationsfest"* bezieht sich hierbei auf die Anforderungen, die eine Hohlfasermembran an Festigkeit aufweisen muss, um in der Verarbeitung der Hohlfasermembran während der Herstellung von Hohlfasermembran und Wellenform unbeschädigt zu bleiben.

In einem dritten Aspekt betrifft die Erfindung einen Hohlfasermembrandialysefilter aufweisend eine Vielzahl von erfindungsgemäßen Hohlfasermembranen.

Der erfindungsgemäße Hohlfasermembrandialysefilter zeichnet sich dadurch aus, dass er hitzesterilisierbar ist, ohne dass die vorteilhaften Trennleistungseigenschaften der erfindungsgemäßen Hohlfasermembranen mit einer Wandstärke von 30 μm oder weniger durch die Hitzesterilisation übermäßig negativ beeinflusst werden. Dadurch kann ein Hohlfasermembrandialysefilter bereitgestellt werden, der hinsichtlich der Clearance gegenüber Hohlfasermembrandialysefiltern mit Hohlfasermembranen aufweisend eine Wandstärke von größer als 30 μm und gleicher effektiver Membranoberfläche verbesserte Eigenschaften aufweist, insbesondere verbesserte Clearance- Eigenschaften aufweist. Weiterhin vorteilhaft ist, dass beim Aufbau eines erfindungsgemäßen Hohlfasermembrandialysefilters bei gleicher Anzahl von Hohlfasermembranen und gleicher effektiver Membranoberfläche durch die reduzierte Wandstärke der erfindungsgemäßen Hohlfasermembranen weniger Membranmaterial benötigt wird als bei Hohlfasermembranen mit größerer Wandstärke. Der erfindungsgemäße Hohlfasermembrandialysefilter kann daher kostengünstiger und unter Einsparung von Rohstoffen gebaut werden. Zudem kann das Filtergehäuse kleiner und platzsparender gestaltet werden.

Der Begriff *"effektive Membranoberfläche"* wird im Sinne der vorliegenden Anmeldung die Oberfläche der Hohlfasermembranen in einem Hohlfasermembrandialysefilter verstanden, die mit der zu filtrierenden Flüssigkeit, insbesondere Blut, in Kontakt steht.

Die Membranoberfläche kann aus den geometrischen Daten der Hohlfasern ermittelt werden.

In einem vierten Aspekt betrifft die Erfindung ein Verfahren zur Herstellung eines Hohlfasermembrandialysefilters nach dem dritten Aspekt der Erfindung dadurch gekennzeichnet, dass das Verfahren die Schritte aufweist:

- Bereitstellen eines Hohlfasermembranbündels, aufweisend eine Vielzahl von Hohlfasermembranen nach wenigstens einer Ausführungsform gemäß des ersten Aspekts der Erfindung oder hergestellt nach wenigstens einer Ausführung gemäß des zweiten Aspekt der Erfindung,
- Bereitstellen eines Filtergehäuses,
- Einbringen des Hohlfasermembranbündels in das Filtergehäuse und Vergießen der Enden der Hohlfasermembranen in dem Filtergehäuse mit einer Vergussmasse,
- Wiedereröffnen der vergossenen Membranenden, so dass ein Fluss durch das Lumen des überwiegenden Teils der Hohlfasern ermöglicht ist, und Endmontieren des Hohlfasermembrandialysefilters,
- Sterilisieren des Hohlfasermembrandialysefilters durch ein Hitzesterilisationsverfahren.

Das erfindungsgemäße Verfahren zur Herstellung eines Hohlfasermembrandialysefilters umfasst die Schritte des Filterbaus und der Hitzesterilisation des Hohlfasermembrandialysefilters. In einer Ausführung sind die Schritte der Herstellung der Hohlfasermembran nach einer Ausführungsform gemäß des zweiten Aspekts der Erfindung diesem Verfahren vorgelagert. Demgemäß werden erfindungsgemäße Hohlfasermembranen nach dem ersten Aspekt der Erfindung oder solche Hohlfasermembranen, die nach einer Ausführungsform gemäß des zweiten Aspekts der Erfindung erhältlich sind zu Hohlfasermembranbündeln zusammengefasst. Die Verfahrensschritte zum Bündeln von Hohlfasermembranen, das Einbringen des Hohlfasermembranbündels in ein Gehäuse eines Hohlfasermembrandialysefilters, das endseitige Vergießen der Hohlfasermembranen in dem Gehäuse und das Wiedereröffnen der vergossenen Membranenden sind bekannte Methoden, die bereits im Massenproduktionsmaßstab angewendet werden. Der Begriff "so dass ein Fluss durch das Lumen des überwiegenden Teils der Hohlfasern ermöglicht ist" ist so zu verstehen, dass mehr als 98% aller Hohlfasern des Hohlfaserbündels nach dem Schritt des Wiedereröffnens gängig sind für die Testflüssigkeiten eines Leistungstests bzw. für Blut in der Dialyseanwendung.

Das Hitzesterilisationsverfahren kann so ausgestaltet sein, dass der erfindungsgemäße Hohlfasermembrandialysefilter mit auf 100 bis 150°C erhitztem Wasser oder Wasserdampf durchspült wird. Der Spülprozess kann insbesondere so geführt werden, dass der erfindungsgemäße Hohlfasermembrandialysefilter und erfindungsgemäße Hohlfasermembranen gleichmäßig erwärmt werden.

In einer weiteren bevorzugten Ausführungsform ist das Verfahren zur Herstellung eines Hohlfasermembrandialysefilters dadurch gekennzeichnet, dass die Behandlung mit Wasser oder Wasserdampf wenigstens einen Schritt umfasst, bei dem Wasser oder Wasserdampf in das Innere der Hohlfasermembran geleitet wird und durch Druckbeaufschlagung durch die Membranwand auf die Außenseite der Fasern permeiert wird. Das Verfahren ist in der DE 102016224627.5 beschrieben und hiermit Bestandteil dieser Anmeldung. Mit Hilfe dieses Verfahrens wird eine besonders sichere Spülung und Hitzesterilisierung bereitgestellt. Zudem wird auf der Dialysatseite das Ausmaß der Zusammenlagerung der Hohlfasern verringert, was den Austausch der Hohlfasermembranen mit dem Dialysat verbessert. Dieser Effekt ist besonders ausgeprägt, wenn erfindungsgemäße Hohlfasern mit geringer Wandstärke von 30 μm und weniger im Zusammenwirken mit einer Ondulationswellenlänge von mehr als 1 mm und weniger als 5 mm, insbesondere weniger als 4 mm, Verwendung finden. Überraschenderweise zeigte sich, dass der Effekt auch bei sehr geringen Amplituden vollständig erhalten bleibt.

In einer weiteren Ausführungsform ist vorgesehen, dass das Verfahren zur Herstellung eines erfindungsgemäßen Hohlfasermembrandialysefilters dadurch gekennzeichnet ist, dass die Hohlfasern bei 100 bis 150°C getrocknet werden. Solchermaßen hohe Trocknungstemperaturen führen zu einem sehr effektiven, zeitoptimierten Trocknungsprozess, ohne dass die Leistungsdaten des erfindungsgemäßen Hohlfasermembrandialysefilters zu stark abfallen. Es kann somit ein effektiv hergestellter Filter mit hohen Leistungsdaten bereitgestellt werden.

**Beispiele und Methoden**

Nachfolgend wird die Erfindung anhand von Beispielen näher erläutert.

Messmethode 1: Bestimmung der Clearance für Natriumionen

Die Bestimmung der Clearance wird an einem nach Beispiel 5 aufgebauten Hohlfasermembrandialysefilter in Anlehnung an die DIN EN ISO 8637:2014 durchgeführt. Als Prüflösung für den Blutbereich wird abweichend von Punkt 5.6.1.2 der Norm eine wässrige Lösung von Natriumchlorid in einer Konzentration von 5 g/l als Prüflösung verwendet. Für den Dialyseflüssigkeitsbereich wird destilliertes Wasser verwendet. Die Natriumionenkonzentrationen werden durch Leifähigkeitsmessungen ermittelt. Auf der Blutseite des Hohlfasermembrandialysefilters wird die Flussrate der Prüflösung auf 300ml/ min eingestellt. Auf der Dialysatseite wird eine Flussrate des destillierten Wassers auf 500 ml/min eingestellt. Die Flüsse der Blut- und Dialysatseiten werden zueinander im Gegenstrom geführt. Die Temperaturen der Flüssigkeiten werden auf 37°C eingestellt.

Messmethode 2: Bestimmung der Porosität

Ein Hohlfasermembranbündel, welches zuvor 2 Stunden bei 105°C im Trockenschrank getrocknet wurde, bestehend aus gleichen Hohlfasermembranen, wird gewogen. Es werden die mittlere Länge der Fasern, der mittlere Innendurchmesser und der mittlere Außendurchmesser und die Anzahl der Fasern bestimmt. Die mittleren Dimensionen werden an mindestens 10 verschiedenen Fasern des Hohlfasermembranbündels ermittelt. Zur Bestimmung der Dimensionen wird bei konstanter Temperatur von 20°C gearbeitet. Aus den Dimensionen wird ein Volumen ermittelt, das durch die Membranwandungen der Hohlfasermembranen des Hohlfasermembranbündels eingenommen wird, indem angenommen wird, dass die Geometrie der Hohlfasermembranen einem Hohlzylinder entspricht. Aus dem ermittelten Volumen und dem gemessenen Gewicht kann die mittlere Dichte der in den Hohlfasermembranen vorliegenden Membranstruktur errechnet werden. Die Porosität in Prozent ergibt sich aus dem Verhältnis von ermittelter zu theoretischer Dichte der Hohlfasermembran bei vollständiger Kompaktheit des Polymeren gemäß folgender Formel:

$$\text{Porosität} = \frac{(\textit{Dichte des kompakten Polymeren} - \textit{gemessene Dichte der Faser})}{\textit{Dichte des kompajkten Polymeren}} * 100$$

Beispiel 1: Herstellung einer erfindungsgemäßen wellenförmigen Hohlfasermembran

Eine Spinnlösung bestehend aus 16 Gewichtsteilen Polysulfon (P3500 der Fa. Solvay), 4,4 Gewichtsteilen Polyvinylpyrrolidon (K82-86 der Fa. Ashland) und 79,6 Gewichtsteilen DMAC werden unter Rühren auf 60°C erwärmt, anschließend entgast und zu einer homogenen Spinnmasse verarbeitet. Die Spinnmasse wird durch eine kreisförmige Ringspaltdüse mit einem zentral geführten Fällmittel, bestehend aus 35% Dimethylacetamid (DMAc) und 65% Wasser, zu einem Spinnfaden extrudiert. Der Ringspalt hat eine Spaltweite von 50 $\mu$m und einen inneren Durchmesser von 200 $\mu$m. Das Fällmittel wird im Inneren des hohlen Spinnfadens geführt. Die Temperatur der Ringspaltdüse beträgt 70°C. Der extrudierte Spinnfaden wird durch einen Fällraum geführt, dessen Atmosphäre eine relative Feuchtigkeit von 100 % aufweist. Die Höhe des Fällspaltes beträgt 200 mm, es wird eine Verweilzeit im Fällspalt von 0,4 sec. eingestellt. Der Spinnfaden wird in ein Fällbad, bestehend aus Wasser eingeleitet, das auf 80°C temperiert ist und zu einer Hohlfasermembran ausgefällt. Anschließend wird die Hohlfasermembran durch Spülbäder, die auf eine Temperatur von 75°C bis 90°C temperiert sind geleitet. Anschließend durchläuft die Hohlfasermembran einen Trocknungsprozess zwischen 100°C und 150°C. Es wird eine Hohlfasermembran mit einer Wandstärke von 25 $\mu$m und einem Lumendurchmesser von 185 $\mu$m erhalten. Die Porosität der Membran beträgt 73%.
Die erhaltene Hohlfasermembran wird anschließend durch ein Ondulationswerkzeug geführt, das zwei ineinandergreifende Zahnräder aufweist. Die Köpfe der Zähne der Zahnräder haben einen Abstand von 2 mm. Die Eingriffstiefe der Zahnräder beträgt 0,150 mm. Das Ondulationswerkzeug ist auf 125°C temperiert. Die erhaltene wellenförmige Hohlfasermembran weist eine Wellenlänge von 2 mm und eine Amplitude von 0,1 mm auf.

Beispiel 2: Herstellung einer erfindungsgemäßen wellenförmigen Hohlfasermembran

Es wird eine Hohlfasermembran nach dem Spinnverfahren gemäß Beispiel 1 hergestellt. Die erhaltene Hohlfasermembran wird durch ein auf 125°C temperiertes Ondulationswerkzeug geführt, wobei die Köpfe der Zähne der Zahnräder einen Abstand von 3 mm aufweisen. Die Eingriffstiefe der Zahnräder beträgt 0,150 mm. Die erhaltene wellenförmige Hohlfasermembran weist eine Wellenlänge von 3 mm und eine Amplitude von 0,1 mm auf.

Beispiel 3: Herstellung einer erfindungsgemäßen wellenförmigen Hohlfasermembran

Es wird eine Hohlfasermembran nach dem Spinnverfahren gemäß Beispiel 1 hergestellt. Die erhaltene Hohlfasermembran wird durch ein auf 125°C temperiertes Ondulationswerkzeug geführt, wobei die Köpfe der Zähne der Zahnräder einen Abstand von 4 mm aufweisen. Die Eingriffstiefe der Zahnräder beträgt 0,150 mm. Die erhaltene wellenförmige Hohlfasermembran weist eine Wellenlänge von 4 mm und eine Amplitude von 0,1 mm auf.

Vergleichsbeispiel 1: Herstellung einer wellenförmigen Hohlfasermembran

Es wird eine Hohlfasermembran nach dem Spinnverfahren gemäß Beispiel 1 hergestellt. Die erhaltene Hohlfasermembran wird durch ein auf 125°C temperiertes Ondulationswerkzeug geführt, wobei die Köpfe der Zähne der Zahnräder einen Abstand von 5 mm aufweisen. Die Eingriffstiefe der Zahnräder beträgt 0,160 mm. Die erhaltene wellenförmige Hohlfasermembran weist eine Wellenlänge von 5 mm und eine Amplitude von 0,1 mm auf.

Vergleichsbeispiel 2: Herstellung einer wellenförmigen Hohlfasermembran

Es wird eine Hohlfasermembran nach dem Spinnverfahren gemäß Beispiel 1 hergestellt. Die erhaltene Hohlfasermembran wird durch ein auf 125°C temperiertes Ondulationswerkzeug geführt, wobei die Köpfe der Zähne der Zahnräder einen Abstand von 8,8 mm aufweisen. Die Eingriffstiefe der Zahnräder beträgt 0,2 mm. Die erhaltene wellenförmige Hohlfasermembran weist eine Wellenlänge von 8,8 mm und eine Amplitude von 0,11 mm auf.

Vergleichsbeispiel 3: Herstellung einer wellenförmigen Hohlfasermembran

Es wird eine Hohlfasermembran nach dem Spinnverfahren gemäß Beispiel 1 hergestellt. Die erhaltene Hohlfasermembran wird durch ein auf 125°C temperiertes Ondulationswerkzeug geführt, wobei die Köpfe der Zähne der Zahnräder einen Abstand von 1 mm aufweisen. Die Eingriffstiefe der Zahnräder beträgt 0,15 mm. Die erhaltene Hohlfasermembran weist Faltungen im Bereich der Ondulationen auf und ist für Flüssigkeiten nur eingeschränkt durchgängig.

Vergleichsbeispiel 4: Herstellung einer wellenförmigen Hohlfasermembran

Es wird eine Hohlfasermembran nach dem Spinnverfahren gemäß Beispiel 1 hergestellt, wobei abweichend eine Wandstärke der Hohlfasermembran von 35 $\mu$m gewählt wird bei gleichem Lumendurchmesser von 185 $\mu$m. Die erhaltene Hohlfasermembran wird durch ein auf 125°C temperiertes Ondulationswerkzeug geführt, wobei die Köpfe der Zähne der Zahnräder einen Abstand von 7,3 mm aufweisen. Die Eingriffstiefe der Zahnräder beträgt 0,3 mm. Die erhaltene wellenförmige Hohlfasermembran weist eine Wellenlänge von 7,2 mm und eine Amplitude von 0,2 mm auf. Die Porosität der Membran beträgt 79,9 %. Die Porosität ist aufgrund der höheren Wandstärke deutlich höher.

Beispiel 4: Herstellung eines erfindungsgemäßen Hohlfasermembrandialysefilters

Die nach den Beispielen 1 bis 3 und den Vergleichsbeispielen 1 bis 3 erhaltenen wellenförmigen Hohlfasermembranen werden von einer Haspel aufgenommen und zu einer Fadenschar zusammengeführt. Aus der gehaspelten Fadenschar werden Hohlfasermembranbündel hergestellt. Dabei werden 9984 Hohlfasermembranen der hergestellten Hohlfasermembranen zu einem Bündel zusammengeführt und in ein Gehäuse eines Hohlfasermembrandialysefilters mit einer einem Innendurchmesser von 28,8 mm eingebracht. Die Länge der Hohlfasermembranen beträgt 279 mm. Die Hohlfasermembranen werden an den Enden in dem Gehäuse des Hohlfasermembrandialysefilters vergossen, so dass in dem aufgebauten Hohlfasermembrandialysefilter ein erster Raum (Blutseite) entsteht, der das Innere der Hohlfasermembranen umfasst und weiterhin ein zweiter Raum (Dialysatseite) entsteht, der den Raum zwischen den Hohlfasermembranen umfasst. Als Vergussmaterial wird Polyurethan der Fa. Elastogran (Polyol C6947 und Isocyanat 136-20) verwendet. Die Vergusshöhe an jedem Bündelende beträgt 22 mm. Die effektive Membranoberfläche des Hohlfasermembrandialysefilters beträgt 1,3 m². Die Ergebnisse der Clearancebestimmung für verschiedene Hohlfasermembrandialysefilter nach Beispiel 4 mit jeweils unterschiedlich gewellten Hohlfasermembranen nach den Beispielen 1 bis 3 und den Vergleichsbeispielen 1 und 2 sind in Tabelle 1 wiedergegeben. Die Messwerte von Vergleichsbeispiel 3 sind nicht ermittelbar. In Vergleichsbeispiel 4 sind folgende Daten abweichend: Für das Hohlfasermembranbündel werden 10752 Hohlfasern verwendet. Es resultiert eine effektive Membranoberfläche von 1,4 m². Dieses Bündel wird in ein Gehäuse mit einem Innendurchmesser von 33,8 mm eingefügt. Die Messwerte des Vergleichsbeispiels 2 sind in Tabelle 2 angegeben.

Beispiel 5: Herstellung eines sterilen Hohlfasermembrandialysefilters

Es werden die nach den Beispielen 1 bis 3 und Vergleichsbeispiel 1 bis 4 erhaltenen wellenförmigen Hohlfasermembranen verwendet um nach Beispiel 4 aufgebaute Hohlfasermembrandialysefilter zu erhalten., Die so erhaltenen Hohlfasermembrandialysefilter werden einer Dampfsterilisation unterzogen. Die Methode der Dampfsterilisation ist in der Anmeldung DE 102016224627.5 detailliert beschrieben. Dazu wird der Hohlfasermembrandialysefilter an eine Sterilisationsapparatur angeschlossen und die Arbeitsschritte gemäß DE 102016224627.5 durchgeführt. Anschließend werden die Hohlfasermembrandialysefilter von der Sterilisationsapparatur entkoppelt und dicht verschlossen. Danach wird an den erhaltenen und sterilisierten Hohlfasermembrandialysefiltern die Clearance für Natriumionen bestimmt. Die Ergebnisse der Clearancebestimmung für verschiedene Hohlfasermembrandialysefilter nach Beispiel 5 mit jeweils unterschiedlich gewellten Hohlfasermembranen nach den Beispielen 1 bis 3 und den Vergleichsbeispielen 1 und 2 sind in Tabelle 1 wiedergegeben. Die Messwerte von Vergleichsbeispiel 3 sind nicht ermittelbar. Das Vergleichsbeispiel 4 ist in Tabelle 2 dargestellt.

Die Tabellen zeigen die erhaltenen Natrium Clearance- Werte, die für Hohlfasermembrandialysefilter nach Beispiel 4 in nicht sterilem und nach Beispiel 5 in sterilem Zustand für Hohlfasermembranen mit Wellenlängen zwischen 2 und 8,8 mm erhalten wurden.

Tabelle 1:

|  | Wellenlänge (mm) | Clearance (ml/min.) unsteril | Clearance (ml/ min.) steril | Differenz (ml/min.) | Wandstärke ($\mu$m) |
|---|---|---|---|---|---|
| Vgl. Bsp. 3 | 1 | - | - | - | 25 |
| Bsp. 1 | 2 | 285 | 278 | 7 | 25 |
| Bsp. 2 | 3 | 285 | 279 | 6 | 25 |
| Bsp. 3 | 4 | 280 | 268 | 12 | 25 |
| Vgl. Bsp. 1 | 5 | 276 | 262 | 14 | 25 |
| Vgl. Bsp. 2 | 8,8 | 269 | 255 | 14 | 25 |

Tabelle 2:

|  | Wellenlänge (mm) | Clearance (ml/min.) unsteril | Clearance (ml/min.) steril | Differenz (ml/min.) | Wandstärke ($\mu$m) |
|---|---|---|---|---|---|
| Vgl. Bsp. 4 | 7,2 | 278 | 265 | 13 | 35 |

Es zeigt sich, dass bei Verwendung von Hohlfasern mit einer Wandstärke von 25 $\mu$m dann eine besonders hohe Clearance von 280 ml/min. oder mehr erzielt wird, wenn die Wellenlänge der Ondulation unter 5 mm gewählt wird. Es zeigt sich weiterhin, dass der Abfall der Clearance- Werte nach der Dampfsterilisation geringer als 13 ml/ min. ist, wenn die Wellenläge unter 5 mm gewählt wird. Es zeigt sich weiterhin, dass der Abfall der Clearance- Werte in einer besonders bevorzugten Ausführungsform nach der Dampfsterilisation geringer als 10 ml/ min. ist, wenn die Wellenläge unter 4 mm gewählt wird. Wird die Wellenlänge zu gering gewählt, was bei einer Länge von 1 mm eingetreten ist, kann kein ausreichender Durchfluss durch den Filter mehr gewährleistet werden, da die Membranen durch Faltungen vorgeschädigt werden.

Aufgrund der höheren Faserdimensionen wurde für Vergleichsbeispiel 4 eine geänderte Faserzahl und ein anderer Gehäusedimension gewählt. Es zeigte sich, dass Vergleichsbeispiel 4 trotz größerer effektiver Membranoberfläche eine deutlich geringere Clearance aufweist, sowohl vor als auch nach der Hitzesterilisierung.

**Patentansprüche**

1. Wellenförmige Hohlfasermembran für die extrakorporale Blutbehandlung, umfassend wenigstens

    ein erstes hydrophobes Polymer, das ausgewählt ist aus der Gruppe der Polyarylether (Polysulfone, Polyarylketone, Polyetherketone), Polyamide, Polyester, Polycarbonate, Polyacrylate und -methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere,
    und wenigstens ein zweites hydrophiles Polymer ausgewählt aus der Gruppe der Polyvinylpyrrolidone oder Polyethylenglykole oder Mischungen hieraus,
    mit einer Wandstärke (w) von 20 $\mu$m oder mehr und 30 $\mu$m oder weniger,
    einer Amplitude (a) der Wellenform im Bereich von 0,005 bis 0,15 mm, insbesondere von 0,01 bis 0,12 mm, und einem Lumendurchmesser (l) von 160 bis 230 $\mu$m,
    **dadurch gekennzeichnet,**

**dass** die Wellenform der Hohlfasermembran eine Wellenlänge ($\lambda$) im Bereich von mehr als 1 mm bis 4 mm oder von mehr als 1 mm und weniger als 4 mm aufweist,
sowie **dadurch gekennzeichnet, dass** die Porosität der Hohlfasermembran größer als 65% und kleiner als 78% ist, wobei die Porosität, wie in der Beschreibung definiert, bestimmt wird.

2. Hohlfasermembran nach Anspruch 1 **dadurch gekennzeichnet, dass** die Wellenform periodisch, insbesondere im Wesentlichen sinusförmig ist.

3. Hohlfasermembran nach einem der vorhergehenden Ansprüche, **dadurch gekennzeichnet, dass** die Porosität der Hohlfasermembran größer als 72% und kleiner als 78%, insbesondere kleiner als 76% ist.

4. Verfahren zur Herstellung einer wellenförmigen Hohlfasermembran für die extrakorporale Blutbehandlung, umfassend wenigstens ein erstes hydrophobes Polymer, ausgewählt aus der Gruppe der Polyarylether (Polysulfone, Polyarylketone, Polyetherketone), Polyamide, Polyester, Polycarbonate, Polyacrylate und- methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere
und wenigstens ein zweites hydrophiles Polymer, welches ausgewählt ist aus der Gruppe der Polyvinylpyrrolidone oder der Polyethylenglykole oder Mischungen hieraus aufweisend die Schritte:

    - Herstellen einer Hohlfasermembran mit einer Wandstärke von 20 $\mu$m oder mehr und 30 $\mu$m oder weniger, einer Porosität, die größer als 65% und kleiner als 78% ist, wobei die Porosität wie in der Beschreibung definiert, bestimmt wird. und einem Lumendurchmesser (I) von 160 bis 230 $\mu$m aus einer Spinnmasse durch einen Spinn- und Phaseninversionsprozess,
    - Bereitstellen wenigstens eines Ondulationswerkzeugs, gegebenenfalls Ausrichten des Ondulationswerkzeugs, zur Erzeugung einer Wellenprägung mit vorbestimmter Wellenlänge und Amplitude,
    - Behandeln der Hohlfasermembran mit dem Ondulationswerkzeugs, so dass eine Wellenlänge der Ondulation von mehr als 1 mm bis 4 mm oder von mehr als 1 mm und weniger als 4 mm und eine Amplitude (a) im Bereich von 0,005 bis 0,15 mm, insbesondere von 0,01 bis 0,12 mm resultiert.

5. Verfahren zur Herstellung einer Hohlfasermembran nach Anspruch 4, **dadurch gekennzeichnet, dass** das Ondulationswerkzeug zwei ineinandergreifende Zahnräder aufweist, zwischen denen die Hohlfasermembran hindurchgeführt wird.

6. Verfahren zur Herstellung einer Hohlfasermembran nach Anspruch 5, **dadurch gekennzeichnet, dass** die Eingriffstiefe der Zähne 0,1 bis 0,5 mm beträgt.

7. Verfahren zur Herstellung einer Hohlfasermembran nach einem der Ansprüche 4 bis 6, **dadurch gekennzeichnet, dass** die Spinnmasse wenigstens ein erstes hydrophobes Polymer, ausgewählt aus der Gruppe der Polyarylether (Polysulfone, Polyarylketone, Polyetherketone), Polyamide, Polyester, Polycarbonate, Polyacrylate und- methacrylate, Polymethacrylimide, Polyvinylidenfluoride, Polyimide oder Polyacrylnitrile oder der Copolymere umfassend entsprechende Monomereinheiten der genannten Polymere oder der Mischungen der genannten Polymere aufweist und wenigstens ein zweites hydrophiles Polymer aufweist, welches ausgewählt ist aus der Gruppe der Polyvinylpyrrolidone oder der Polyethylenglykole oder Mischungen hieraus und wenigstens ein Lösungsmittel, insbesondere N-Methylpyrrolidon, N,N-Dimethylacetamid, N,N-Diemthylformamid oder Dimethylsulfoxid aufweist.

8. Hohlfasermembrandialysefilter aufweisend eine Vielzahl von Hohlfasermembranen nach einem der Ansprüche 1 bis 3 oder aufweisend eine Vielzahl von Hohlfasermembranen, hergestellt nach einem Verfahren gemäß der Ansprüche 4 bis 7.

9. Verfahren zur Herstellung eines Hohlfasermembrandialysefilters nach Anspruch 8, aufweisend die Schritte

    - Bereitstellen eines Hohlfasermembranbündels, aufweisend eine Vielzahl von Hohlfasermembranen nach wenigstens einem der Ansprüche 1 bis 3 oder hergestellt nach wenigstens einem der Ansprüche 4 bis 7,
    - Bereitstellen eines Filtergehäuses,
    - Einbringen des Hohlfasermembranbündels in das Filtergehäuse und Vergießen der Enden der Hohlfasermembranen in dem Filtergehäuse mit einer Vergussmasse,
    - Wiedereröffnen der vergossenen Membranenden, so dass ein Fluss durch das Lumen des überwiegenden Teils der Hohlfasern ermöglicht ist, und Endmontieren des Dialysefilters,

- Sterilisieren des Hohlfasermembrandialysefilters durch ein Hitzesterilisationsverfahren.

**10.** Verfahren zur Herstellung eines Hohlfasermembrandialysefilters nach Anspruch 9, **dadurch gekennzeichnet, dass** das Hitzesterilisationsverfahren ein Schritt umfasst, bei dem der Hohlfasermembrandialysefilter mit auf 100 bis 150°C erhitztem Wasser oder Wasserdampf durchspült wird.

**11.** Verfahren zur Herstellung eines Hohlfasermembrandialysefilters nach Anspruch 10, **dadurch gekennzeichnet, dass** die Behandlung mit Wasser oder Wasserdampf wenigstens einen Schritt umfasst, bei dem Wasser oder Wasserdampf in das Innere der Hohlfasermembranen geleitet wird und durch Druckbeaufschlagung durch die Membranwand auf die Außenseite der Fasern permeiert wird.

**12.** Verfahren zur Herstellung eines Hohlfaserdialysemembranfilters nach einem der Ansprüche 9 bis 11, **dadurch gekennzeichnet, dass** die Hohlfasermembranen bei einer Temperatur von 100 bis 150°C getrocknet werden.

**Claims**

**1.** An undulated hollow fiber membrane for extracorporeal blood treatment, comprising at least

a first hydrophobic polymer selected from the group of polyaryl ethers (polysulfones, polyaryl ketones, polyether ketones), polyamides, polyesters, polycarbonates, polyacrylates and -methacrylates, polymethacrylimides, polyvinylidene fluorides, polyimides or polyacrylonitriles or copolymers comprising corresponding monomer units of said polymers or mixtures of said polymers,
and at least one second hydrophilic polymer selected from the group of polyvinylpyrrolidones or polyethylene glycols or mixtures thereof,
having a wall thickness (w) of 20 $\mu$m or more and 30 $\mu$m or less,
an amplitude (a) of the waveform in the range from 0.005 to 0.15 mm, in particular from 0.01 to 0.12 mm, and
a luminal diameter (l) of 160 to 230 $\mu$m,
**characterized in that**
that the waveform of the hollow fiber membrane has a wavelength ($\lambda$) in the range from more than 1 mm to 4 mm or from more than 1 mm and less than 4 mm,
and **characterized in that** the porosity of the hollow fiber membrane is greater than 65% and less than 78%, wherein the porosity is determined as defined in the description.

**2.** A hollow fiber membrane according to any one of the preceding claims 1, **characterized in that** the waveform is periodic, in particular substantially sinusoidal.

**3.** A hollow fiber membrane according to any one of the preceding claims, **characterized in that** the porosity of the hollow fiber membrane is greater than 72% and less than 78%, in particular less than 76%.

**4.** A method for producing an undulated hollow fiber membrane for extracorporeal blood treatment, comprising at least a first hydrophobic polymer selected from the group of polyarylethers (polysulfones, polyaryl ketones, polyetherketones), polyamides, polyesters, polycarbonates, polyacrylates and methacrylates, polymethacrylimides, polyvinylidene fluorides, polyimides or polyacrylonitriles or copolymers comprising corresponding monomer units of said polymers or mixtures of said polymers

and at least one second hydrophilic polymer selected from the group of polyvinylpyrrolidones or polyethylene glycols or mixtures thereof,
comprising the steps:

- producing a hollow fiber membrane having a wall thickness of 20 $\mu$m or more and 30 $\mu$m or less, a porosity greater than 65% and less than 78%, the porosity being determined as defined in the description, and a luminal diameter (l) of 160 to 230 $\mu$m from a spinning mass by a spinning and phase inversion process,
- providing at least one undulation tool, optionally orientating the undulation tool, in order to produce a waveform of predetermined wavelength and amplitude,
- processing the hollow fiber membrane with the undulation tool, so as to yield an undulation wavelength of more than 1 mm to 4 mm or of more than 1 mm and less than 4 mm and an amplitude (a) in the range from 0.005 to 0.15 mm, in particular from 0.01 to 0.12 mm.

**5.** The method of producing a hollow fiber membrane according to claim 4, **characterized in that** the undulation tool comprises two intermeshing gears between which the hollow fiber membrane is passed.

**6.** The method of producing a hollow fiber membrane according to claim 5, **characterized in that** the depth of engagement of the teeth is 0.1 to 0.5 mm.

**7.** The method for producing a hollow fiber membrane according to any one of claims 4 to 6, **characterized in that** the spinning mass comprises at least a first hydrophobic polymer selected from the group of polyarylethers (polysulfones, polyaryl ketones, polyetherketones), polyamides, polyesters, polycarbonates, polyacrylates and methacrylates, polymethacrylimides, polyvinylidene fluorides, polyimides or polyacrylonitriles or copolymers comprising corresponding monomer units of said polymers or mixtures of said polymers, and at least one second hydrophilic polymer selected from the group consisting of polyvinylpyrrolidones or polyethylene glycols or mixtures thereof and comprising at least one solvent, in particular N-methylpyrrolidone, N,N-dimethylacetamide, N,N-diemthylformamide or dimethyl sulfoxide.

**8.** A hollow fiber membrane dialyzer comprising a plurality of hollow fiber membranes according to any one of claims 1 to 3 or comprising a plurality of hollow fiber membranes produced by a process according to claims 4 to 7.

**9.** A method for producing a hollow fiber membrane dialysis filter according to claim 8, comprising the steps of:

- providing a hollow fiber membrane bundle comprising a plurality of hollow fiber membranes according to at least one of claims 1 to 3 or made according to at least one of claims 4 to 7,
- providing a filter housing,
- introducing the hollow fiber membrane bundle into the filter housing and potting the ends of the hollow fiber membranes in the filter housing with a potting compound,
- reopening the potted membrane ends so as to enable a flow through the lumen of most of the hollow fibers and final assembly of the dialysis filter,
- sterilizing the hollow fiber membrane dialysis filter by a heat sterilization process.

**10.** The method for producing a hollow fiber membrane dialyzer according to claim 9, **characterized in that** the heat sterilization process comprises a step in which the hollow fiber membrane dialyzer is flushed with water or vapor heated to 100 to 150°C.

**11.** The method of producing a hollow fiber membrane dialyzer according to claim 10, **characterized in that** the water or vapor treatment comprises at least one step in which the water or water vapor is conducted into the interior of the hollow fiber membranes and permeates through the membrane wall to the exterior of the fibers under the application of pressure.

**12.** The method of producing a hollow fiber membrane dialyzer according to any one of claims 9 to 11, **characterized in that** the hollow fiber membranes are dried at a temperature of 100 to 150°C.

**Revendications**

**1.** Membrane en fibre creuse de forme ondulée pour le traitement extracorporel du sang, comprenant au moins

un premier polymère hydrophobe, qui est choisi dans le groupe des polyaryléthers (polysulfones, polyarylcétones, polyéthercétones), polyamides, polyesters, polycarbonates, polyacrylates et polyméthacrylates, polyméthacrylamides, polyvinylidène fluorures, polyimides ou polyacrylonitriles ou des copolymères comprenant des unités monomériques correspondantes des polymères cités ou des mélanges des polymères cités, et au moins un deuxième polymère hydrophile choisi dans le groupe des polyvinylpyrrolidones ou polyéthylène glycols ou des mélanges de ceux-ci, avec une épaisseur de paroi (w) de 20 μm ou plus et de 30 μm ou moins, une amplitude (a) de la forme ondulée dans la plage de 0,005 à 0,15 mm, en particulier de 0,01 à 0,12 mm, et un diamètre de lumière (1) de 160 à 230 μm, **caractérisée en ce que** la forme ondulée de la membrane en fibre creuse présente une longueur d'onde (X) dans la plage allant de plus de 1 mm à 4 mm ou de plus de 1 mm et de moins de 4 mm,

et **caractérisée en ce que** la porosité de la membrane en fibre creuse est supérieure à 65 % et inférieure à 78 %, la porosité étant déterminée telle que définie dans la description.

2. Membrane en fibre creuse selon la revendication 1, **caractérisée en ce que** la forme ondulée est périodique, en particulier sensiblement sinusoïde.

3. Membrane en fibre creuse selon l'une des revendications précédentes, **caractérisée en ce que** la porosité de la membrane en fibre creuse est supérieure à 72 % et inférieure à 78 %, notamment inférieure à 76 %.

4. Procédé de fabrication d'une membrane en fibre creuse de forme ondulée pour le traitement extracorporel du sang, comprenant au moins un premier polymère hydrophobe, choisi dans le groupe des polyaryléthers (polysulfones, polyarylcétones, polyéthercétones), polyamides, polyesters, polycarbonates, polyacrylates et polyméthacrylates, polyméthacrylamides, polyvinylidène fluorures, polyimides ou polyacrylonitriles ou des copolymères comprenant des unités monomériques correspondantes des polymères cités ou des mélanges de polymères cités,

et au moins un deuxième polymère hydrophile qui est choisi dans le groupe des polyvinylpyrrolidones ou des polyéthylène glycols ou des mélanges de ceux-ci,
comportant les étapes suivantes :

- fabriquer une membrane en fibre creuse avec une épaisseur de paroi de 20 $\mu$m ou plus et de 30 $\mu$m ou moins, une porosité qui est supérieure à 65 % et inférieure à 78 %, la porosité étant déterminée telle que définie dans la description, et un diamètre de lumière (1) de 160 à 230 $\mu$m à partir d'une masse de filage grâce à un processus de filage et d'inversion de phases,
- préparer au moins un outil d'ondulation, le cas échéant orienter l'outil d'ondulation, pour produire une empreinte ondulée ayant une longueur d'onde et une amplitude prédéfinies,
- traiter la membrane en fibre creuse avec l'outil d'ondulation de telle sorte qu'il en résulte une longueur d'onde de l'ondulation de plus de 1 mm à 4 mm ou de plus de 1 mm et de moins de 4 mm, et une amplitude (a) dans la plage de 0,005 à 0,15 mm, en particulier de 0,01 à 0,12 mm.

5. Procédé de fabrication d'une membrane en fibre creuse selon la revendication 4, **caractérisé en ce que** l'outil d'ondulation présente deux roues dentées engrenées, entre lesquelles la membrane en fibre creuse est introduite.

6. Procédé de fabrication d'une membrane en fibre creuse selon la revendication 5, **caractérisé en ce que** la profondeur de mise en prise des dents est de 0,1 à 0,5 mm.

7. Procédé de fabrication d'une membrane en fibre creuse selon l'une des revendications 4 à 6, **caractérisé en ce que** la masse de filage présente au moins un premier polymère hydrophobe, choisi dans le groupe des polyaryléthers (polysulfones, polyarylcétones, polyéthercétones), polyamides, polyesters, polycarbonates, polyacrylates et polyméthacrylates, polyméthacrylamides, polyvinylidène fluorures, polyimides ou polyacrylonitriles ou des copolymères comprenant des unités monomériques correspondantes des polymères cités ou des mélanges des polymères cités, et au moins un deuxième polymère hydrophile qui est choisi dans le groupe des polyvinylpyrrolidones ou des polyéthylène glycols ou des mélanges de ceux-ci et au moins un solvant, en particulier la N-méthylpyrrolidone, le N,N-diméthylacétamide, le N,N-diméthylformamide ou le diméthylsulfoxyde.

8. Filtre de dialyse à membrane en fibre creuse présentant une pluralité de membranes en fibre creuse selon l'une des revendications 1 à 3 ou présentant une pluralité de membranes en fibre creuse, fabriquées par un procédé selon les revendications 4 à 7.

9. Procédé de fabrication d'un filtre de dialyse à membrane en fibre creuse selon la revendication 8, présentant les étapes suivantes

- préparer un faisceau de membranes en fibre creuse présentant une pluralité de membranes en fibre creuse selon au moins l'une des revendications 1 à 3 ou fabriquées selon au moins l'une des revendications 4 à 7,
- préparer un boîtier de filtre,
- introduire le faisceau de membranes en fibre creuse dans le boîtier de filtre et couler les extrémités des membranes en fibre creuse dans le boîtier de filtre avec une masse de coulée,
- rouvrir les extrémités de membrane coulées de telle sorte qu'un flux puisse passer à travers la lumière de la partie prépondérante des fibres creuses et terminer de monter le filtre de dialyse,

- stériliser le filtre de dialyse à membrane en fibre creuse grâce à un procédé de stérilisation à la chaleur.

10. Procédé de fabrication d'un filtre de dialyse à membrane en fibre creuse selon la revendication 9, **caractérisé en ce que** le procédé de stérilisation à la chaleur comprend une étape lors de laquelle le filtre de dialyse à membrane en fibre creuse est rincée avec de l'eau chauffée à une température de 100 à 150 °C ou de la vapeur d'eau.

11. Procédé de fabrication d'un filtre de dialyse à membrane en fibre creuse selon la revendication 10, **caractérisé en ce que** le traitement avec de l'eau ou de la vapeur d'eau comprend au moins une étape lors de laquelle de l'eau ou de la vapeur d'eau est introduite dans l'intérieur des membranes en fibre creuse et est infiltrée par perméation par application de pression à travers la paroi de la membrane contre la face externe des fibres.

12. Procédé de fabrication d'un filtre de dialyse à membrane en fibre creuse selon l'une des revendications 9 à 11, **caractérisé en ce que** les membranes en fibre creuse sont séchées à une température de 100 à 150 °C.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1685862 A1
- EP 1671695 A1
- EP 2253371 A1
- JP S57194007 B
- EP 2659914 A1
- JP 2010036127 A
- JP 2006051094 A
- DE 102016224627